# EUROPEAN PATENT APPLICATION

(11) **EP 4 644 551 A1**
(43) Date of publication of application: **05.11.2025**
(21) Application number: 23911949.8
(22) Date of filing: 21.12.2023
(51) Int. Cl.: C12N 15/53, A01H 5/00, C12N 1/15, C12N 1/21, C12N 5/10, C12N 9/04, C12N 15/63, C12N 15/82, C12P 19/00

(54) **2-HYDROXYFLAVANONE C-GLYCOSIDE DEHYDRATASE GENE AND USE THEREOF**

(30) Priority: 28.12.2022 JP 2022212502
(71) Applicant: Suntory Holdings Limited, Osaka-shi, Osaka 530-8203 (JP)
(72) Inventor: TANAKA, Yoshikazu, Soraku-gun, Kyoto 619-0284 (JP); NAKAMURA, Noriko, Soraku-gun, Kyoto 619-0284 (JP); TAKAFUJI, Kazuaki, Soraku-gun, Kyoto 619-0284 (JP)
(74) Representative: Vossius & Partner Patentanwälte Rechtsanwälte mbB
(86) International application number: PCT/JP2023/045990
(87) International publication number: WO 2024/143169

(57) **Abstract**

Provided is a transgenic plant that has improved stress resistance and/or modified flower color, and modification to a blue flower color, or its inbred or outbred progeny, or their propagules, partial plant bodies, tissue or cells, as well as a method which allows easy and efficient production of flavone C-glycoside. A polynucleotide encoding an enzyme with dehydrating activity for 2-hydroxyflavanone C-glycoside is used.

## Description

### FIELD

The present invention relates to a polynucleotide coding for an enzyme with dehydrating activity for 2-hydroxyflavanone C-glycoside, and to its use.

### BACKGROUND

Flavonoids, as typical plant secondary metabolites, have phenylalanine as the starting substance, and they are classified into numerous groups such as flavanones, flavones, isoflavone flavonols and anthocyanins, depending on the structure of the C-ring. The enzymes involved in their biosynthesis, and genes coding for the enzymes, have been well researched. Isoflavones and flavones are both biosynthesized from flavanones. For an isoflavone, isoflavone synthase catalyzes hydroxylation at position 2 of a flavanone and transfer of the B-ring (NPL 1), and then 2-hydroxyisoflavone dehydratase further acts to produce an isoflavone (NPL 2). Isoflavone synthase belongs to the cytochrome P450 family, while 2-hydroxyisoflavone dehydratase belongs to the carboxy esterase family. Flavones are present in numerous plants as aglycones, C-glycosides and O-glycosides (NPL 3). For example, plants of the family Caryophyllaceae, genus *Dianthus,* such as carnation (*Dianthus caryophyllus*) and nadeshiko (*Dianthus* sp.) produce flavone O-glycosides and flavone C-glycosides (NPL 4, NPL 5 and NPL 6). Flavone C-glycosides are also produced by gentian, horseradish, buckwheat and Poaceae plants. The functions of flavones in plants include protection from ultraviolet rays, interaction with microorganisms, protection from biological stress by production of phytoalexins, and blueing of anthocyanins by copigment action. For example, flavone C-glycosides such as isovitexin function as copigments for irises and gene recombinant carnations, producing blue flower color (NPL 7 and NPL 8).

Flavones are synthesized from flavanones by two routes (NPL 3). Flavone synthases catalyze the reaction for conversion of flavanones to flavones. For flavone synthases, genes coding for flavone synthase I belonging to the dioxygenase family (NPL 9) and flavone synthase II belonging to the cytochrome P450 family (NPL 10) are known. The produced flavones are metabolized into flavone O-glycosides or flavone C-glycosides by UDP-glucose-dependent glycosyltransferase. For example, the flavone O-glycosyltransferase gene of nemophila (NPL 11) and the flavone C-glycosyltransferase gene (NPL 12, NPL 13) of gentian and horseradish have already been isolated. Another route is via 2-hydroxyflavanone as an intermediate, where 2-hydroxyflavanone is produced first by the catalytic action of flavanone 2-hydroxylase (NPL 14). A 2-hydroxyflavanone C-glycoside is then produced by the action of glycosyltransferase at the 2-position hydroxyl group. This glycosyltransferase gene has already been reported (NPL 15). The 2-hydroxyflavanone C-glycoside is converted to a mixture of flavone 6-C-glycosides and flavone 8-C-glycosides (typically isovitexin and vitexin) by spontaneous dehydration reaction *in vitro* (NPL 15, NPL 16).

In the body, the dehydrating reaction is believed to be catalyzed by 2-hydroxyflavanone C-glycoside dehydratase. While such enzyme activity has been reportedly detected in rice leaves (NPL 17), the enzyme itself and its related gene have been unknown. As in the isoflavone biosynthesis described above it has been suggested that enzymes belonging to the carboxy esterase family can potentially catalyze the same dehydrating reaction (NPL 18), but no enzyme activity has been exhibited, and the possibility remains as a conjecture. Moreover, the dehydratase has not been purified nor has its gene been isolated, while there is also no example of its industrial use.

### [CITATION LIST]

### [NON PATENT LITERATURE]

[NPL 1] Plant Physiol. 1999 121:821-828
[NPL 2] Plant Physiol 2005 137:882-91
[NPL 3] Phytochemistry 2005 66:2399-2407
[NPL 4] Z. Naturforsch. 63c, 161D168 (2008)
[NPL 5] Chem. Pharm. Bull. 59(9)1141-1148(2011)
[NPL 6] Phytochemistry. 2003 May; 63(1):15-23
[NPL 7] Plant Physiol Biochem. 2013 72:116-24
[NPL 8] Phytochemistry. 2003; 63:15-23
[NPL 9] Phytochemistry 2001 58:43-6
[NPL 10] Plant Cell Physiol. 1999 40:1182-6
[NPL 11] Plant Cell Physiol. 2018 59:2075-2085
[NPL 12] FEBS Lett. 2015 589:182-7
[NPL 13] Plant Cell Physiol. 2019 Dec 1; 60(12):2733-2743
[NPL 14] FEBS Lett. 1998 Jul 17; 431(2):287-90
[NPL 15] J Biol Chem. 2009 3; 284:17926-34
[NPL 16] Metabolic Engineering 2013 16:11-20
[NPL 17] J Biol Chem. 2009 3; 284:17926-34
[NPL 18] Journal of Agricultural and Food Chemistry 2019 67(40), 11262-11276

### SUMMARY

### [TECHNICAL PROBLEM]

It is an object of the present invention to provide a transgenic plant that has improved stress resistance and/or modified flower color, preferably modification to a blue flower color, or its inbred or outbred progeny, or their propagules, partial plant bodies, tissue or cells, as well as a method for easily and efficiently producing flavone C-glycoside.

### [SOLUTION TO PROBLEM]

The present inventors have discovered 2-hydroxyflavanone C-glycoside dehydratase activity in petals of Caryophyllaceae plants, and have succeeded in purifying the enzyme from petals of Caryophyllaceae plants. As a result of further diligent research, the present inventors have succeeded in obtaining a gene coding for 2-hydroxyflavanone C-glycoside dehydratase derived from Caryophyllaceae plants, and the invention has been completed upon this finding.

Specifically, the present invention provides the following.
[1] A polynucleotide encoding an enzyme with dehydrating activity for 2-hydroxyflavanone C-glycoside, wherein the polynucleotide is selected from the group consisting of the following (A) to (E):
   (A) polynucleotides consisting of the nucleotide sequences of SEQ ID NO: 3, 5, 7 or 9;
   (B) polynucleotides that hybridize with polynucleotides consisting of nucleotide sequences complementary to the nucleotide sequences of SEQ ID NO: 3, 5, 7 or 9 under stringent conditions;
   (C) polynucleotides encoding proteins consisting of the amino acid sequences of SEQ ID NO: 4, 6, 8 or 10;
   (D) polynucleotides encoding proteins consisting of the amino acid sequences of SEQ ID NO: 4, 6, 8 or 10 having a deletion, substitution, insertion and/or addition of one or more amino acids; and
   (E) polynucleotides encoding proteins having amino acid sequences with at least 90% identity to the amino acid sequences of SEQ ID NO: 4, 6, 8 or 10.
[2] A polynucleotide according to [1], which is a polynucleotide consisting of the nucleotide sequence of SEQ ID NO: 3, 5, 7 or 9.
[3] A polynucleotide according to [1], which is a polynucleotide encoding a protein consisting of the amino acid sequence of SEQ ID NO: 4, 6, 8 or 10.
[4] A protein encoded by a polynucleotide according to any one of [1] to [3].
[5] A vector comprising a polynucleotide according to any one of [1] to [3].
[6] A transgenic plant, or its inbred or outbred progeny, that comprises a polynucleotide according to any one of [1] to [3].
[7] Propagules, partial plant bodies, tissue or cells of a transgenic plant according to [6] or its inbred or outbred progeny.
[8] Cut flowers of a transgenic plant according to [6], or its inbred or outbred progeny, or a processed form created from the cut flowers.
[9] A method for creating a transgenic plant,
   the method comprising a step of introducing into plant cells a polynucleotide according to any one of [1] to [3].
[10] A non-human host containing a polynucleotide according to any one of [1] to [3].
[11] A method for producing an enzyme with dehydrating activity for 2-hydroxyflavanone C-glycoside, the method comprising:
   a step of culturing a non-human host according to [10], and
   a step of harvesting from the non-human host an enzyme with dehydrating activity for 2-hydroxyflavanone C-glycoside.
[12] A method for producing flavone C-glycoside,
   wherein the method includes contacting an enzyme produced by the method according to [11] with 2-hydroxyflavanone C-glycoside.

### [ADVANTAGEOUS EFFECTS OF INVENTION]

According to the invention it is possible to provide a transgenic plant that has improved stress resistance and/or modified flower color, and modification to a blue flower color, or its inbred or outbred progeny, or their propagules, partial plant bodies, tissue or cells. According to the invention it is also possible to provide a non-human host (for example, yeast or bacteria) that expresses an enzyme with dehydrating activity for 2-hydroxyflavanone C-glycoside, and to use it to easily and efficiently produce flavone C-glycoside.

### BRIEF DESCRIPTION OF DRAWINGS

Fig. 1 shows the biosynthetic pathway for flavone C-glycoside in plants, via 2-hydroxyflavanone as an intermediate.
Fig. 2 shows high-performance liquid chromatograms for a crude protein solution extract from *E. coli* expressing Dca32587.1 derived from carnation, and of an enzyme reaction solution obtained by enzyme reaction on 2-hydroxynaringenin 6-C-glucoside.
Fig. 3 shows high-performance liquid chromatograms for a crude protein solution extract from *E. coli* expressing both DN21819 derived from *Silene* and *Osteospermum* KAH9622207.1, and for an enzyme reaction solution obtained by enzyme reaction on 2-hydroxynaringenin 6-C-glucoside. Also shown is a carnation-derived Dca32587.1-derived protein solution, as a control.
Fig. 4 shows high-performance liquid chromatograms for a crude protein solution extract from *E. coli* expressing full-length cDNA for dehydrogenase derived from a nadeshiko variety (SEQ ID NO: 9), and for an enzyme reaction solution obtained by enzyme reaction on 2-hydroxynaringenin 6-C-glucoside.

Plant flowers and leaves contain flavones. Flavones are a typical type of plant secondary metabolite commonly known as flavonoids, as cyclic ketones of flavan derivatives which are mainly found as glycosides in plants. Flavone, in the strict definition, refers to 2,3-didehydroflavan-4-one, which is a compound with chemical formula C₁₅H₁₀O₂ and molecular weight 222.24, but in the wider sense flavones are a category of flavonoids, a flavonoid being classified as a "flavone" if it has a flavone structure as the basic backbone and also lacks the hydroxyl group at the 3-position.

In plants, flavones are also distributed as glycosides, in addition to the free form, such that flavone O-glycosides and flavone C-glycosides are mainly produced, but flavone C-glycosides in particular are known to exhibit powerful copigment action, with blue color being exhibited by intermolecular interaction in the co-presence of anthocyanins (a pigment group abundantly found in plants), and especially delphinidin-type anthocyanins such as delphinidin, malvidin and petunidin. Copigment action includes not only a color depth effect that induces blue color production, but also a deep color effect or an effect of increasing color stability. Flavones have the function of imparting to plants various biological or non-biological stress resistances, as phytoalexins, or contributing to establishment of interaction with microorganisms.

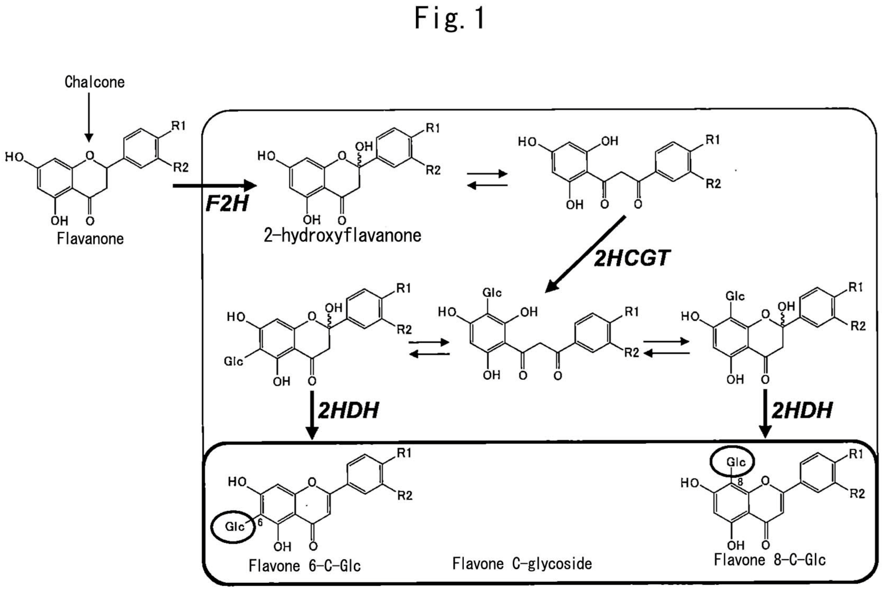

As used herein, "flavone C-glycoside" means a glycoside of a flavone in the wide sense, i.e. a derivative falling under the definition of flavones, wherein an aglycone is directly bonded to the anomeric carbon of an aldose. Flavone C-glycosides include, but are not limited to, luteolin C-glycoside, tricetin C-glycoside, apigenin C-glycoside and acacetin C-glycoside. Flavone C-glycosides also include glycosides of apigenin, luteolin, tricetin and acacetin derivatives. Two routes are known for the biosynthetic pathway of flavone C-glycosides in plants. Of these, flavone 6-C-glucoside and flavone 8-C-glucoside are produced by the action of flavanone 2-hydroxylase (F2H), flavone C-glycosylase (2HCGT) and dehydratase (2HDH) in a pathway via 2-hydroxyflavanone as the intermediate (Fig. 1). It is therefore conjectured that by incorporating the 2HDH gene, one of the essential genes of this pathway, into a host plant, it might be possible to accumulate flavone C-glycoside in plant cells.

The present inventors have succeeded in acquiring a polynucleotide encoding an enzyme with dehydrating activity for 2-hydroxyflavanone C-glycoside from petals of Caryophyllaceae plants. The present invention relates to a novel polynucleotide coding for an enzyme having dehydrating activity for 2-hydroxyflavanone C-glycoside.

Enzymes having dehydrating activity for 2-hydroxyflavanone C-glycoside, to be used for the invention, will typically be derived from Caryophyllaceae plants, examples of Caryophyllaceae plants including carnation (*Dianthus caryophyllus*), *Silene littorea, Heliosperma pusillum* and nadeshiko gardening species (*Dianthus hybrida*), with no limitation to these, however.

A polynucleotide of the invention is preferably selected from the group consisting of the following (A) to (E):
(A) polynucleotides consisting of the nucleotide sequences of SEQ ID NO: 3, 5, 7 or 9;
(B) polynucleotides that hybridize with polynucleotides consisting of nucleotide sequences complementary to the nucleotide sequences of SEQ ID NO: 3, 5, 7 or 9 under stringent conditions;
(C) polynucleotides encoding proteins consisting of the amino acid sequences of SEQ ID NO: 4, 6, 8 or 10;
(D) polynucleotides encoding proteins consisting of the amino acid sequences of SEQ ID NO: 4, 6, 8 or 10 having a deletion, substitution, insertion and/or addition of one or more amino acids; and
(E) polynucleotides encoding proteins having amino acid sequences with at least 90% identity to the amino acid sequences of SEQ ID NO: 4, 6, 8 or 10.

SEQ ID NO: 3 is the nucleotide sequence of a gene coding for an enzyme (Dca32587.1) having dehydrating activity for 2-hydroxyflavanone C-glycoside derived from carnation, SEQ ID NO: 5 is the nucleotide sequence of a gene coding for an enzyme (DN21819) having dehydrating activity for 2-hydroxyflavanone C-glycoside derived from *Silene littorea,* SEQ ID NO: 7 is an enzyme (KAH9622207.1) having dehydrating activity for 2-hydroxyflavanone C-glycoside derived from *Heliosperma pusillum,* and SEQ ID NO: 9 is the nucleotide sequence of a gene coding for an enzyme having dehydrating activity for 2-hydroxyflavanone C-glycoside derived from the nadeshiko variety Miite.

The term "polynucleotide" as used herein refers to DNA or RNA.

Also as used herein, the term "stringent conditions" refers to conditions that allow specific binding between a polynucleotide or oligonucleotide and genomic DNA in a selective and detectable manner. Stringent conditions are defined by an appropriate combination of salt concentration, organic solvent (for example, formamide), temperature and other known conditions. Specifically, stringency is increased by reducing the salt concentration, increasing the organic solvent concentration or raising the hybridization temperature. Stringency is also affected by the rinsing conditions after hybridization. The rinsing conditions are defined by the salt concentration and temperature, and stringency of rinsing is increased by reducing the salt concentration and raising the temperature. Therefore, the term "stringent conditions" means conditions such that specific hybridization takes place only between nucleotide sequences with high identity, such as a degree of "identity" between the nucleotide sequences of about 80% or greater, preferably about 90% or greater, more preferably about 95% or greater, even more preferably 97% or greater and most preferably 98% or greater, on average. The "stringent conditions" may be, for example, a temperature of 60°C to 68°C, a sodium concentration of 150 to 900 mM and preferably 600 to 900 mM, and a pH of 6 to 8, with specific examples including hybridization under conditions of 5 × SSC (750 mM NaCl, 75 mM trisodium citrate), 1% SDS, 5 × Denhardt solution, 50% formaldehyde, 42°C, and rinsing under conditions of 0.1 × SSC (15 mM NaCl, 1.5 mM trisodium citrate), 0.1% SDS, 55°C.

The hybridization may be carried out by a method that is publicly known in the field or a similar method, such as the method described in Current Protocols in Molecular Biology (edited by Frederick M. Ausubel et al., 1987). When a commercially available library is to be used, the hybridization may be carried out according to the method described in the accompanying directions for use. The gene selected by hybridization may be naturally derived, such as plant-derived or non-plant-derived. The gene selected by the hybridization may be cDNA, genomic DNA or chemically synthesized DNA.

SEQ ID NO: 4 is the amino acid sequence of an enzyme (Dca32587.1) having dehydrating activity for 2-hydroxyflavanone C-glycoside derived from carnation, SEQ ID NO: 6 is the amino acid sequence of an enzyme (DN21819) having dehydrating activity for 2-hydroxyflavanone C-glycoside derived from *Silene littorea,* SEQ ID NO: 8 is an enzyme (KAH9622207.1) having dehydrating activity for 2-hydroxyflavanone C-glycoside derived from *Heliosperma pusillum,* and SEQ ID NO: 10 is the amino acid sequence of an enzyme having dehydrating activity for 2-hydroxyflavanone C-glycoside derived from the nadeshiko variety Miite.

The phrase "amino acid sequence having a deletion, substitution, insertion and/or addition of one or more amino acids" means an amino acid sequence having a deletion, substitution, insertion and/or addition of 1 to 20, preferably 1 to 5 and more preferably 1 to 3 arbitrary amino acids. Site-directed mutagenesis is a useful genetic engineering method as it allows introduction of specific mutations into specified sites, and it may be carried out by the method described in Molecular Cloning: A Laboratory Manual, 2nd Ed., Cold Spring Harbor Laboratory Press, Cold Spring Harbor, N.Y., 1989. By expressing the mutant DNA using a suitable expression system, it is possible to obtain a protein consisting of an amino acid sequence having a deletion, substitution, insertion and/or addition of one or more amino acids. A polynucleotide can be obtained by a method that is publicly known to those skilled in the art, such as a method of chemical synthesis using the phosphoramidite method, or a nucleic acid amplification method using a plant nucleic acid specimen as template, and primers designed based on the nucleotide sequence of the target gene.

Throughout the present specification, the term "identity" means, for polypeptide sequences (or amino acid sequences) or polynucleotide sequences (or nucleotide sequences), the quantity (number) of amino acid residues or nucleotides composing them that can be determined to be identical between two chains, in the sense of mutual agreement between them, meaning the degree of sequence correlation between two polypeptide sequences or two polynucleotide sequences, and this "identity" can be easily calculated. Numerous methods are known for measuring identity between two polynucleotide sequences or polypeptide sequences, and the term "identity" is well known to those skilled in the art (for example, see Lesk, A.M. (Ed.), Computational Molecular Biology, Oxford University Press, New York, (1988); Smith, D.W. (Ed.), Biocomputing: Informatics and Genome Projects, Academic Press, New York, (1993); Grifin, A.M. & Grifin, H.G. (Ed.), Computer Analysis of Sequence Data: Part I, Human Press, New Jersey, (1994); von Heinje, G., Sequence Analysis in Molecular Biology, Academic Press, New York (1987); Gribskov, M. & Devereux, J. (Ed.), Sequence Analysis Primer, M-Stockton Press, New York, (1991) and elsewhere).

Also, the numerical values for "identity" used in the present specification, unless otherwise specified, may be the numerical values calculated using an identity search program known to those skilled in the art, but they are preferably numerical values calculated using the ClustalW program of MacVector Application (version 9.5, Oxford Molecular Ltd., Oxford, England). According to the invention, the degree of "identity" between amino acid sequences is, for example, about 65% or greater, about 70% or greater, about 75% or greater, about 80% or greater, about 85% or greater or about 90% or greater, preferably about 95% or greater, even more preferably about 97% or greater, even more preferably about 98% or greater and most preferably about 99% or greater.

The polynucleotide (nucleic acid, gene) of the invention "encodes" a protein of interest. The term "encodes" also includes both encoding a structural sequence (exon) that is a continuous section of the protein of interest, and encoding via an intervening sequence (intron). Also, the term "encodes" includes both encoding a structural sequence (exon) that is a continuous section of the protein of interest, and encoding via an intervening sequence (intron).

A gene with a natural nucleotide sequence can be obtained by analysis using a DNA sequencer, for example. DNA encoding an enzyme having a modified amino acid sequence can be synthesized using common site-specific mutagenesis or PCR, based on DNA having the natural nucleotide sequence. For example, a DNA fragment to be modified may be obtained by restriction enzyme treatment of natural cDNA or genomic DNA, and used as template for site-specific mutagenesis or PCR using primers with the desired mutation, to obtain a DNA fragment having the desired modification. The DNA fragment having the mutation may then be linked with a DNA fragment encoding another portion of the target enzyme.

Alternatively, in order to obtain DNA encoding an enzyme consisting of a shortened amino acid sequence, it is possible to cut DNA encoding an amino acid sequence longer than the target amino acid sequence, such as the full-length amino acid sequence, with a desired restriction enzyme, and if the obtained DNA fragment does not code for the full target amino acid sequence, then a DNA fragment consisting of the sequence of the missing portion may be synthesized and linked to it.

By expressing the obtained polynucleotide using a gene expression system in *Escherichia coli* or yeast and measuring the enzyme activity, it is possible to confirm that the obtained polynucleotide encodes a protein with the desired activity.

The present invention relates to a (recombinant) vector, and especially an expression vector, comprising the aforementioned polynucleotide. The vector of the invention also comprises an expression control region, such as a promoter, terminator and replication origin, that are dependent on the type of host plant into which it is introduced. Examples of promoters that constitutively express polynucleotides in plant cells include cauliflower mosaic virus 35S promoter, El₂35S promoter having two 35S promoter enhancer regions linked together, and the rd29A gene promoter, rbcS promoter and mac-1 promoter. For tissue-specific gene expression, a promoter for a gene expressed specifically in that tissue may be used.

The vector may be created by a common method using a restriction enzyme and ligase. Transformation of a host plant using the expression vector may also be carried out by a common method.

The invention further relates to a transgenic plant containing the aforementioned polynucleotide or vector, or to its inbred or outbred progeny.

At the current level of technology for creation of transgenic plants, it is possible to use techniques to introduce a polynucleotide into a plant and constitutively or tissue-specifically express the polynucleotide. Transfer of the DNA into the plant may be carried out by a method known to those skilled in the art, such as the *Agrobacterium* method, binary vector method, electroporation method, PEG method or particle gun method.

Plants to be used as hosts for the invention are not particularly restricted and may be plants belonging to the genus Rosaceae *Rosa,* Solanaceae *Petunia,* Compositae *Chrysanthemum,* Caryophyllaceae *Dianthus* or Liliaceae *Lilium,* among which rose cultivar of Rosaceae *Rosa* (scientific name: *Rosa hybrida*) is especially preferred. The term "rose plant", as used herein, is a rose cultivar of Rosaceae *Rosa* (scientific name: *Rosa hybrida*), which is its taxonomical classification. Roses are largely classified as Hybrid Tea, Floribunda and Polyantha roses based on their tree form and flower size, with the major pigment (anthocyanin) in the petals of all lines being of two types, the cyanidin-type and pelargonidin-type. The type of rose plant used as a host for the invention is not particularly restricted, and any of these varieties or lines are suitable. Examples of rose varieties to be used as hosts include Ocean Song, Noblesse, Rita Perfumera, Cool Water, Fame, Topless and Peach Avalanche.

By introducing a polynucleotide of the invention into plant cells, the flavone C-glycoside accumulates in the plant cells and acts as a phytoalexin, thereby improving the stress resistance and exhibiting copigment action in the co-presence of delphinidin-type anthocyanins, and allowing creation of a transgenic plant with a flower color modified to blue flower color, or its inbred or outbred progeny.

The invention still further relates to cut flowers of the obtained transgenic plant or its inbred or outbred progeny, or the propagules, partial plant body, tissue or cells, or a processed form created from the cut flowers (especially processed cut flowers). The processed cut flowers referred to here include pressed flowers formed using cut flowers, or preserved flowers, dry flowers or resin sealed products, with no limitation to these.

The invention also relates to a non-human host comprising the aforementioned polynucleotide or vector. The non-human host used may be a prokaryote or eukaryote. A prokaryote may be a common host, such as a bacterium belonging to the genus *Escherichia,* such as *Escherichia coli,* or a *Bacillus* microorganism such as *Bacillus subtilis.* Eukaryotes used may be lower eukaryotic organisms such as eukaryotic microorganisms, examples of which are fungi such as yeasts or filamentous fungi as fungi. Examples of yeasts include *Saccharomyces* microorganisms such as *Saccharomyces cerevisiae,* and as filamentous fungi, *Aspergillus* microorganisms such as *Aspergillus oryzae, Aspergillus niger* and *Penicillium* microorganisms. A non-human host used may also be animal cells or plant cells, where animal cells used may be cell lines from mice, hamsters, monkeys or humans, and insect cells, such as silkworm cells or the silkworms themselves, may also be used as the host.

The invention still further relates to a method for producing an enzyme with dehydrating activity for 2-hydroxyflavanone C-glycoside, the method comprising a step of incorporating a polynucleotide of the invention into a non-human host, a step of culturing the non-human host, and a step of harvesting from the non-human host an enzyme with dehydrating activity for 2-hydroxyflavanone C-glycoside, and the method still further relates to a method of producing flavone C-glycoside, as well as to a method of contacting an enzyme produced by the method with 2-hydroxyflavanone C-glycoside. This method allows easy and efficient production of flavone C-glycosides.

The present invention will now be explained in greater detail by examples.

### EXAMPLES

### [Example 1: Detection of flavone C-glycosides in nadeshiko petals]

It has previously been reported that nadeshiko petals contain flavone O-glycosides and flavone C-glycosides. Pigment analysis was carried out to confirm whether or not the target flavone C-glycoside was detected in the petals of the commercial product nadeshiko variety (NP) and large-violet (BNG) tested here.

After freezing nadeshiko petal samples, they were dried overnight with a VirTis sentry 2.0 vacuum freezing drier (SP SCIENTIFIC), and then gently crushed with a spatula. To the crushed sample there was added 4 mL of 0.1% trifluoroacetic acid (TFA)-containing 50% acetonitrile per 0.5 g of fresh weight, and after treatment with ultrasonic waves for 20 minutes, the mixture was centrifuged at 3,600 rpm, 4°C, 10 minutes, and the supernatant was recovered. The obtained supernatant was filtered with a 0.45 µm filter (COSMO NICE filter (aqueous), 0.45 µm, 13 mm). A 200 µL portion was dried, and after addition of β-glucosidase and naringinase it was treated overnight at 30°C, and 200 µL of 0.1% TFA-containing 90% acetonitrile was added to suspend the reaction. It was then treated for 2 minutes with ultrasonic waves, after which it was centrifuged at 15,000 rpm, 4°C, 5 minutes, and the obtained supernatant was filtered with a 0.45 µm filter (Milex-LH, 0.45 µm, Millipore) and provided for high-performance liquid chromatography. The analysis conditions were as follows.

### <Analysis conditions>

Apparatus: Prominence HPLC system (product of Shimadzu Corp.)
Detector: SPD-M20A (250 to 450 nm)
Column: Shim-pack FC-ODS 150 x 4.6 mm, 3 µm (Shimadzu GLC)
Eluent A: 0.1% TFA aqueous solution
Eluent B: 0.1% TFA-containing 90% acetonitrile
Flow rate: 0.6 mL/min

Elution was performed using solution A (0.1% TFA aqueous solution) and solution B (aqueous 90% acetonitrile solution containing 0.1% TFA). The elution was with a 20-minute linear concentration gradient from a 9:1 mixture to a 8:2 mixture, a 15-minute linear concentration gradient from a 8:2 mixture to a 2:8 mixture and a 5-minute linear concentration gradient from a 2:8 mixture to a 0:10 mixture, followed by elution with a 0:10 mixture for 1 minute. The flow rate was 0.6 mL/min. As a result of analysis by the method described above, isovitexin and isoorientin were detected from NP, while isovitexin alone as a flavone C-glycoside was detected from BNG.

**[Table 1]**

| Variety | Flavone C-glycoside (mg content per 1 g fresh weight) | |
|---|---|---|
| | Isovitexin | Isoorientin |
| NP | 0.961 | 0 |
| BNG | 0.097 | 0.874 |

### [Example 2: Measurement of dehydratase activity for 2-hydroxyflavanone C-glycoside in crude enzyme extract of nadeshiko petals]

Approximately 1 g of nadeshiko petals (NP and BNG) was pulverized into a powder form using a mortar and pestle in the presence of liquid nitrogen. To this there was added 15 ml of extraction buffer A (0.1 M tris-HCl buffer pH 7.5, 1 mM dithiothreitol (DTT), 1/100 dilution protease inhibitor cocktail (general use, 100-fold concentration), Nacalai Tesque, Inc.), and the protein was extracted on ice. This was centrifuged at 10,000 rpm for 10 minutes at 4°C, and the supernatant was collected. The volume of the obtained supernatant was measured, ammonium sulfate was added to 35% saturation, and then the mixture was slowly stirred at 4°C for 1.5 hours. It was centrifuged at 10,000 rpm for 10 minutes at 4°C, and the supernatant was collected. The volume of the obtained supernatant was measured, ammonium sulfate was added to 70% saturation, and then the mixture was slowly stirred at 4°C for 3 hours. After centrifugal separation at 10,000 rpm, 4°C for 10 minutes, the supernatant was discarded and the precipitate was suspended in 400 µL of extraction buffer B (20 mM Tris-HCl buffer at pH 7.5, 1 mM DTT, 1/100 dilution protease inhibitor cocktail (general use, 100-fold concentration)). The mixture was centrifuged at 10,000 rpm, 4°C for 10 minutes, the obtained supernatant was desalted with MicroSpin G-25 Columns (Cytiva) according to the method recommended by the manufacturer, and the protein fraction was recovered.

Enzyme reaction was conducted for 30 minutes at 30°C with a 50 µl reaction mixture comprising 2-hydroxynaringenin 6-C-glucoside (which can be prepared by the method described in Metabolic Engineering 2013 16:11-20, for example) at a final concentration of 200 µM, 100 mM potassium phosphate buffer pH 7.5, 10% sucrose, 1 mM DTT, and 32.5 µl of a crude enzyme solution. After stopping the reaction by addition of 50 µl of 90% methanol, the mixture was centrifuged at 15,000 rpm, 4°C, 5 minutes, and the obtained supernatant was filtered with a 0.45 µm filter (Milex-LH, 0.45 µm, Millipore) and provided for high-performance liquid chromatography. The analysis conditions were as follows.

### <Analysis conditions>

Apparatus: LC-2030 High-performance liquid chromatograph (Shimadzu Corp.)
Column: Shim-pack FC-ODS 150 x 4.6 mm, 3 µm (Shimadzu GLC)
Eluent A: 0.1% formic acid aqueous solution
Eluent B: 0.1% formic acid-containing methanol
Flow rate: 1.0 mL/min

Solution A (0.1% formic acid aqueous solution) and solution B (0.1% formic acid-containing methanol) were used for elution. The elution was with a 12-minute linear concentration gradient from a 9:1 mixture to a 4:6 mixture of both, holding for 8 minutes with the 4:6 mixture, subsequent 1-minute linear concentration gradient from a 4:6 mixture to a 0:10 mixture of both, and then holding for 1 minute with the 0:10 mixture. The flow rate was 1 mL/min. Under these conditions, the 2-hydroxynaringenin 6-C-glucoside substrate elutes at approximately 8.7 minutes, vitexin elutes at approximately 10.6 minutes, and isovitexin elutes at approximately 11.1 minutes. As a result of analysis by the method described above, new reaction compound peaks were detected in both of the reaction solutions of NP and BNG, matching the retention time and absorption spectrum of the isovitexin sample. It was thus shown that an enzyme is present in the nadeshiko petals that catalyzes dehydrating reaction from 2-hydroxynaringenin 6-C-glucoside to isovitexin.

### [Example 3: Isolation of candidate genes coding for proteins catalyzing dehydration reaction of 2-hydroxyflavanone C-glycoside]

### <Isolation of total RNA>

Using an RNeasy Plant Mini Kit (Qiagen), the total RNA was isolated from nadeshiko petals (variety: Miite Raspberry Rose, Peach Latte) and leaves (variety: Miite Raspberry Rose), according to the manufacturer's recommended protocol. The petals were classified on a 5-level scale by growth. Variety Miite Peach Latte was used only in Level 5.
Level 1: Petals completely enclosed by calyx
Level 2: Tips of petals slightly exposed from calyx
Level 3: Tips of petals exposed at about 1 mm from calyx
Level 4: Approximately 1/3 of petals exposed from calyx
Level 5: At least half of petals exposed from calyx

### <Expression analysis of nadeshiko-derived cDNA>

An RNA-Seq library was prepared from the total RNA prepared as described above, with different respective tags, using a SureSelect Strand-Specific RNA library preparation kit (Agilent Technologies) according to the manufacturer's recommended protocol. 150 base length paired-end sequences were obtained using a NextSeq next-generation sequencer (Illumina). The reads from all of the samples were then combined and assembled using a Trinity v2.12.0, and 415,080 contig sequences were obtained. The contigs that had not been mapped by RSEM 1.3.0 were removed, and 94,257 contig sequences were obtained as valid transcription products. When variants were present in each gene the longest sequence was selected, and 46,267 contig sequences were finally obtained. The pair reads for each sample were mapped, and the FPKM value was calculated to determine the expression level.

### <Estimation of gene function>

The gene functions of the obtained contig sequences were estimated based on a DIAMOND search in the NCBI NR database and a BLAST search in the Araport11.

### <Acquiring full-length cDNA of candidate gene>

Of the obtained contig sequences, a BLAST search was conducted using the amino acid sequence of the *Glycyrrhiza echinata*-derived 2-hydroxyisoflavanone dehydratase gene (GeHIDM, NCBI Accession: AB154414.1) or a keyword search was carried out using "Dehydratase", and 9 different types of genes (DN226, DN601, DN1155, DN4559, DN5671, DN9033, DN1313, DN1658, DN1010) were selected from among the obtained candidate genes having high expression levels and being mainly expressed in the petals. Full-length cDNA clones were obtained by artificial gene synthesis based on the assembled full-length cDNA sequence. Since the sequence of DN601 obtained by assembly was of incomplete length, a gene corresponding to the gene was searched in a carnation database (http://carnation.kazusa.or.jp/), to obtain Dca57486.1.

### [Example 4: Measurement of enzyme activity in E. coli by protein having dehydrating activity for 2-hydroxyflavanone C-glycoside]

### <Construction of E. coli expression vector>

The 9 candidate genes described above were used as candidates to prepare *E. coli* expression vectors containing their full lengths using pET15b (Novagen), according to the manufacturer's recommended protocol.

### <Expression in E. coli>

Each prepared *E. coli* expression vector was introduced into the *E. coli* strain BL21(DE3) (New England Biolabs Japan Inc) by the manufacturer's recommended protocol, to obtain *E. coli* transformants. The *E. coli* were cultured using an Overnight Express Autoinduction System 1 (Novagen), according to the manufacturer's recommended protocol. The *E. coli* transformants were cultured at 37°C (approximately 4 hours) with 2 mL of prepared culture solution, to an OD600 value of 0.5. The *E. coli* solution was added as a preculturing solution to 50 mL of culture solution, and main culturing was carried out overnight at 25°C. The *E. coli* solution that had been cultured by main culturing overnight was centrifuged at 3000 rpm, 4°C, 15 minutes, and the collected cells were suspended in sonic buffer (20 mM Tris-HCl buffer at pH 7.5,10 mM DTT, 10 µM amidinophenylmethanesulfonyl fluoride HCl (APMSF)). After adding 5 mL of sonic buffer to 1 g of *E. coli,* the suspended *E. coli* was disrupted by ultrasonic treatment and then centrifuged at 15,000 rpm, 4°C, 10 minutes, and the supernatant was recovered. The supernatant was used as a crude protein solution extract from *E. coli* expressing the candidate gene.

### <Enzyme activity measurement>

Enzyme reaction was conducted for 30 minutes at 30°C with a 50 µl reaction mixture comprising 2-hydroxynaringenin 6-C-glucoside at a final concentration of 40 µM, 100 mM potassium phosphate buffer pH 7.5, 10% sucrose, 14 µM β-mercaptoethanol and 33.6 µl of crude enzyme solution. After stopping the reaction by addition of 50 µl of 90% methanol, the mixture was centrifuged at 15,000 rpm, 4°C, 5 minutes, and the obtained supernatant was filtered with a 0.45 µm filter (Milex-LH, 0.45 µm, Millipore), provided for high-performance liquid chromatography, and analyzed by the same method as Example 2. As a result, no new reaction compound peak was detected in any of the reaction mixtures, nor was any dehydratase activity found for 2-hydroxynaringenin 6-C-glucoside. It has therefore not been possible to obtain target genes based on similarity with previously reported amino acid sequences and gene function estimation.

**[Table 2]**

| Candidate gene | Activity for 2HNCG |
|---|---|
| DN226 | - |
| DN1155 | - |
| DN4559 | - |
| DN5671 | - |
| DN9033 | - |
| DN1313 | - |
| DN1658 | - |
| DN1010 | - |
| Dca57486.1 | - |

### [Example 5: Measurement of dehydratase activity of 2-hydroxyflavanone C-glycoside]

Enzyme reaction was conducted for 1 to 3 hours at 30°C with a 20 µl reaction mixture comprising 2-hydroxyflavanone 6-C-glucoside at a final concentration of 200 µM, 20 mM potassium phosphate buffer pH 7.0 and 1-10 µl of crude enzyme solution. After then adding 20 µl of acetonitrile to suspend the reaction, 160 µl of 50% acetonitrile was added, the mixture was centrifuged at 10,000 rpm for 15 minutes at 25°C, the supernatant was filtered, and then the reaction product was detected by HPLC (LC-2030C by Shimadzu Corp.) under the following conditions. Column: Shim-pack FC-ODS 4.6 x 150 mm, solvent A: 0.1% formic acid, solvent B: methanol containing 0.1% formic acid. The mobile phase is a linear concentration gradient from A:B = 90:10 at 0 minutes to A:B = 40:60 at 12 minutes, a linear concentration gradient of A:B = 40:60 up to 20 minutes and A:B = 0:100 for 21 minutes, and after holding for 1 minute, a linear concentration gradient up to A:B = 90:10. The flow rate was 1 ml/min, and the column temperature was 40°. The absorption spectrum was measured as the absorbance at 280 nm and 330 nm using a photodiode array (SPD-M20A). Under these conditions, the 22-hydroxyflavanone 6-C-glucoside substrate elutes at about 8.6 minutes, vitexin elutes at about 10.6 minutes, and isovitexin elutes at about 11.1 minutes.

### [Example 6: Database construction for genes expressed in petals]

The database constructed from the nadeshiko petals is shown in Example 3. Also, RNAseq data (NCBI Accession: PRJNA299760 ID:299760) for petals of *Silene littorea* of the Caryophyllaceae family was used for assembly using Trinity v2.12.0 to create contig sequences, to obtain a transcription library for *Silene littorea.*

### [Example 7: Enzyme purification from nadeshiko variety NP]

After freezing 25 g of pre-opening petals of the nadeshiko variety NP with liquid nitrogen, they were crushed into powder with a mortar and pestle. To this there were added 200 mL of 20 mM potassium phosphate buffer at pH 7.0, 100 mM sodium chloride and 1 mM DTT, and extraction was performed for 40 minutes with a stirrer while cooling with ice. The extract was centrifuged with a 4°-cooled centrifuge at 6000 rpm for 15 minutes, and the supernatant was collected and filtered with a Miracloth. After dissolving ammonium sulfate in the filtrate to 35% saturation, it was centrifuged in the same manner. Ammonium sulfate was then dissolved in the supernatant to 65% saturation, and the mixture was again centrifuged. The precipitate was dissolved in a minimum amount of 20 mM potassium phosphate buffer at pH 7.0, and dialysis was performed for 16 hours against 20 mM potassium phosphate buffer at pH 7.0 containing 0.1 mM DTT. The precipitate was removed by centrifugal separation and adsorbed onto 10 ml of Q-Sepharose Fast Flow (Cytiva). After washing the column with 35 ml of 20 mM potassium phosphate buffer at pH 6.2, it was further washed with 50 mM sodium chloride-containing 20 mM potassium phosphate buffer at pH 7.0. The protein was then eluted with 300 mM sodium chloride-containing 20 mM potassium phosphate buffer at pH 7.0, and about 1 ml each was recovered. The activity of each fraction was measured as in Example 5. The fractions with strong activity for conversion of the substrate to isovitexin were collected, and ammonium sulfate was dissolved to 1 M.

The solution was charged into a column with 10 ml of Butyl Sepharose Fast Flow (Cytiva), equilibrated with 20 mM potassium phosphate buffer at pH 7.0 and 1 M ammonium sulfate, and washed with 30 ml of the same buffer. It was then eluted with 20 mM potassium phosphate buffer at pH 7.0 and 0.6 M ammonium sulfate, and about 1 ml each was recovered. The activity of each fraction was measured in the manner described above, and the fractions where strong activity was seen for conversion of the substrate to isovitexin were collected and concentrated to about 0.6 ml using Amicon Ultra10K (Merck-Millipore). The concentrate was supplied for gel filtration chromatography with Superdex ^{™}75 10/300GL (Cytiva). The solvents used were 50 mM potassium phosphate, pH 7.0, and 150 mM sodium chloride, with a flow rate of 0.8 ml/min and 1 ml/fraction (using AKTA pure, Cytiva).

The fraction of the active peak was diluted 5-fold with water and charged into 1 ml of Hi Trap DEAE FF (Cytiva) that had been equilibrated with 20 mM potassium phosphate buffer at pH 7.0. Using a flow rate of 1 ml/min, washing was performed with 20 mM potassium phosphate buffer at pH 7.0 for 10 minutes, and followed by elution with a 30-minute linear concentration gradient up to 20 mM potassium phosphate buffer at pH 7.0 + 300 mM sodium chloride, and 0.3 ml each was collected (using AKTA pure).

The fractions with strong activity were diluted 3-fold with water and adsorbed onto 0.5 g of 40 µM CHT Ceramic Hydroxyapatite, Type 1 (BioRad) that had been equilibrated with 5 mM potassium phosphate, pH 7.0. The mixture was washed with 5 ml of the same buffer, further washed with 5 ml of 20 mM potassium phosphate, pH 7.0 and washed with 5 ml of 40 mM potassium phosphate, pH 7.0, and eluted with 120 mM potassium phosphate, pH 7.0. Approximately 400 µl of each was recovered. The active fraction was diluted 5-fold with water and adsorbed onto 1 ml of Hi Trap Q FF (Cytiva). Using a flow rate of 1 ml/min, washing was performed with the same buffer for 5 minutes at 0.25 ml/tube, and followed by elution with a 10-minute linear concentration gradient up to 20 mM potassium phosphate, pH 7.0 + 1 M sodium chloride (using AKTA pure).

The activity of each tube was measured, and the following analysis was conducted.

In order to obtain peptide fragments from the proteins in each tube, enzyme digestion was carried out by the Phase transfer surfactant (PTS) method (see J Proteome Res. 2008 7:731-740, for example). The fragmented peptide sample solution was measured by mass spectrometry using a nanoLC-MS system having an UltiMate 3000 Nano nanoflow chromatograph (Thermo Fisher Scientific) and an LTQ Orbitrap Fusion Lumos high-precision mass spectrometer (Thermo Fisher Scientific) connected online. As conditions for the nanoLC, the peptide sample was injected and adsorbed onto a C18 reversed-phase column at a flow rate of 10 µl/min, and separated at a flow rate of 300 nl/min with a linear gradient using solvent A (3% acetonitrile/0.1% formic acid aqueous solution) and solvent B (90% acetonitrile/0.1% formic acid aqueous solution). Peptide was then sprayed from a nanoelectrospray ion source (Thermo Fisher Scientific) and the generated precursor ion was introduced into a mass spectrometer for MS measurement. The precursor ion was fragmented by Collision Induced Dissociation (CID) to produce product ion, which was measured by MS/MS. The normalized collision energy value for CID was set to 32. The MS spectrum and MS/MS spectrum were obtained with setting for automatic switching from MS to MS/MS, in data-dependent acquisition mode. The MS scan was obtained with Orbitrap at a half-width resolution of 300,000, and the subsequent MS/MS scan was obtained with an ion trap. Each measurement was in automated gain control (AGC) mode, and the AGC value was set to 1 × 10⁶ for MS and 1 × 10⁴ for MS/MS.

Proteome Discoverer version 2.5 (PD) was used for analysis of the MS/MS spectrum obtained in LC-MS. The CID scan data were compared with the protein sequence database from nadeshiko RNAseq mentioned in Example 2, using the SEQUEST-HT search algorithm. Trypsin was set as the protease, and the mass tolerance was 10 ppm for the precursor ion and 0.02 Da for the fragment ions. Oxidation of methionine (M) (+15.995 Da) was set as a variable modification, and carbamidomethylation of cysteine (C) (+57.021 Da) was set as a fixed modification. The false discovery rate (FDR) of the peptide was calculated with PD, based on 1.0% FDR. Numerous sequences correlating with relative abundance based on activity level and mass spectrum intensity were observed, such as DN48015_c0_g1_i5.

### [Example 8: Enzyme purification from nadeshiko variety BNG]

A mortar and pestle were used to pulverize 130 g of nadeshiko petals into powder, in the presence of liquid nitrogen. To this there were added 600 ml of 20 mM potassium phosphate buffer at pH 7.0, 100 ml sodium chloride and 400 µM DTT, and the mixture was stirred with a stirrer for 2 hours on ice to extract the protein. The mixture was centrifuged at 8000 rpm for 20 minutes, and the supernatant was filtered with a Miracloth and collected. After adding 150 ml of water to the precipitate, it was suspended and then centrifuged at 8000 rpm for 20 minutes, after which the supernatant was filtered with a Miracloth and collected, and added to the previous collected solution.

After adding ammonium sulfate to 35% saturation for dissolution, the solution was centrifuged at 8000 rpm for 20 minutes and the supernatant was collected. Ammonium sulfate was added to the mixture to 70% saturation, and the supernatant was discarded. The precipitate was dissolved in about 40 ml of 20 mM potassium phosphate buffer at pH 7.0, and dialyzed against 10 mM potassium phosphate buffer at pH 7.0 + 0.1 mM DTT. After dialysis, centrifugal separation was carried out at 6000 rpm for 10 minutes, and the supernatant was adsorbed onto two 20 ml Q-Sepharose Fast Flow platforms, at half each. Each was washed with 20 ml of potassium phosphate buffer at pH 7.0, 20 mM potassium phosphate buffer at pH 6.2, 50 ml of potassium phosphate buffer at pH 7.0, and 50 mM sodium chloride. Elution was subsequently carried out with 30 ml of potassium phosphate buffer at pH 7.0 and 200 mM sodium chloride, and then 30 ml of potassium phosphate buffer at pH 7.0 and 500 mM sodium chloride. Approximately 3 ml of eluate was collected each. The activity of each fraction was measured, and the fraction with observed activity was dialyzed against 10 mM potassium phosphate buffer at pH 7.0.

The dialyzed fraction was adsorbed onto 18 ml of DEAE Sepharose Fast Flow that had been equilibrated with the same buffer. The product was washed with 20 ml of 10 mM potassium phosphate buffer at pH 7.0, 20 ml of 10 mM potassium phosphate buffer at pH 7.0, 20 mM sodium chloride, 20 ml of 10 mM potassium phosphate buffer at pH 7.0, 100 mM sodium chloride, 20 ml of 10 mM potassium phosphate buffer at pH 7.0, and 200 mM sodium chloride, in that order. The fraction with observed activity was adsorbed onto 6 g of CHT Ceramic Hydroxyapatite, Type 1 that had been equilibrated with 10 mM of 40 µM potassium phosphate, pH 7.0. After washing with 30 ml of 40 mM potassium phosphate, pH 7.0, it was eluted with 120 mM potassium phosphate, pH 7.0. The fraction with detected activity was diluted 5-fold with water and then adsorbed onto HiTrap Sepharose Q Fast Flow. The protein was eluted using 20 mM potassium phosphate buffer at pH 7.0 as base, with a linear concentration gradient from 0 mM to 1 M (total: 75 ml, flow rate: 2 ml/min), and recovered at 1.5 ml each. The active fraction was separated into a first half (relatively weak adsorption onto HiTrap Sepharose Q, as fraction A) and a second half (relatively strong adsorption on HiTrap Sepharose Q, as fraction B), and each was fractionated in the following manner.

After dissolving ammonium sulfate in each to 1 M, it was adsorbed onto 6 ml of Butyl Sepharose Fast Flow that had been equilibrated with 20 mM potassium phosphate buffer at pH 7.0 + 1 M ammonium sulfate. After washing with about 25 ml of the same solution, elution was performed with 20 mM potassium phosphate buffer pH 7.0 + 0.6 M ammonium sulfate. After collecting 2 ml each, the active fractions were concentrated to 0.5 ml using Amicon Ultra 30K (Merck Millipore). This was followed by gel filtration chromatography in the same manner as Example 7, and each active fraction with strongest activity was supplied for subsequent purification.

After 5-fold dilution with water, column chromatography was carried out using 1 ml of Hi Trap DEAE FF (Cytiva), as described in Example 7. The activity due to fraction A was eluted at the same holding time as Example 7. However, the activity due to fraction B had a peak of activity at a location with a long retention time, suggesting the possibility that this was due to two enzyme molecule species with different isoelectric points.

The active fractions derived from fractions A and B were adsorbed onto 0.5 g of 40 µM CHT Ceramic Hydroxyapatite, Type 1 that had been equilibrated with 20 mM potassium phosphate buffer at pH 7.0. After washing with 3 ml of 50 mM potassium phosphate buffer at pH 7.0, it was eluted with 3 ml of 100 mM potassium phosphate buffer at pH 7.0 and then eluted with 3 ml of 140 mM potassium phosphate buffer at pH 7.0. About 0.4 ml of eluate was collected each. The activity was eluted with 140 mM potassium phosphate buffer at pH 7.0 for the sample derived from fraction A, and with both 100 mM and 140 mM potassium phosphate buffer at pH 7.0 for the sample derived from fraction B. The proteins in each fraction were analyzed by MS to identify the corresponding nadeshiko contigs. The proteins with parallel activity and MS intensity were thought to be derived from the nucleotide sequences DN48015_c0_g1_i5 and DN44046_c2_g1_i1 prepared in Example 3.

After fractionation of the samples from fractions A and B using hydroxyapatite, the fraction with the highest activity was further fractionated by isoelectric gel electrophoresis using Isoelectric-Focusing Novex (Thermo Fisher Science) at pH 3-7. A portion of the lane was stained with a silver staining kit (FujiFilm-Wako Pure Chemical Industries), two gels corresponding to the bands were collected from fraction A and two from fraction B, and extraction was performed at 4°C for 48 hours with 0.4 ml of 20 mM potassium phosphate buffer at pH 7.0 + 0.1% Triton X-100. After recovery, the mixture was concentrated with Amicon Ultra 30K and the activity was measured. MS analysis of the protein was carried out in the same manner as described above. In this analysis as well, DN44046_c2_g1_i1 was detected as a contig exhibiting MS intensity correlating with activity.

### [Example 9: Identification of candidate genes]

The nucleotide sequence of DN44046_c2_g1_i1 is as follows.

TCAGTTCAGCTCACTTCAGTTCAGTTCAGCACAGCTATATTATTTAATAGAACTTCAT
TCAGTTCAGCTCAGCTCAATTCAGTTCAGTTCAGCTCATTTCAGTTCAGTTCAGCTGT
AAAAAGTCCGAAAGAACAGCCATTTTTCGGGACAGGAGGCAGAAACCCAGAATCAT
TCCTAAATGAATTCAGCACTGTAGTTCTTCTAACTTCCTATAATACGTCATATGAAA

GTCAACACAAGATCTTTGCTCAGAAGAAGGGGGTGATAGTGAAGGCGACTAAAGAT
CAAATCACAGCTATGACAGCAGAAATCGAACAAGAGAAAGAGAAGAACAAATCAA
CGACGAC (SEQ ID NO: 1)

The nucleotide sequence of DN48015_c0_g1_i5 is as follows.

TCAACAGGTAATATATTTGTCGACATTACGCTCGCTAATCTCTATATTTTTGTCAAAA
ACTTGCTTTAGATGTAGCTAATTGAAGGTACCTATAGGATTAGTGTGACCCTGTAAG
CAAATCGGTTTGTCGACATTTTTGACGCTTGTCTATGTTCAGTGTATAAAAAATTTTC
TTGTGACTCAATATGTGTGTGTAATTTTATAGGGTGGCATGAACACTCCGTTTATTAG
TTCAAGGGCAACAAAGATCATAATGGTAGTAGACGGTCGAGGCCGCCTCGAGATGC
CGGCCTTAAACGATAGCATAGATGGCACAACCGAAAACTATTACAAGCAAGTCAGC
ACGGAACTAAAGCCGGGTATGGTGTTTGTCGTTCCTCCCGGCCATCCTATGGTTCTA
ATGTCTTCTCAGGACGAAAAACTCGAAACTATTTGCTACGAAGTGAATGCCAAAGG
AAATCAAAAGTTTTCCATCGCAGGGAAAGTC (SEQ ID NO: 2)

Since these nucleotide sequences were both short and therefore unlikely to be the full length, a search was conducted for genes corresponding to them in a carnation database (http://carnation.kazusa.or.jp/). The carnation gene Dca32587.1 exhibited 96% identity with DN44046_c2_g1_i1, and 99% identity with DN48015_c0_g1_i5. In other words, this suggested that DN44046_c2_g1_i1 and DN48015_c0_g1_i5 are fragments from the same gene.

The nucleotide sequence of the open reading frame of the carnation gene Dca32587.1 consists of 1161 bp including the terminal codon.

ATGGGTGAAAATGGAATAAAAAATGAGAGTAACACTTGTTATGTGTTCAATGATGA
AGATTTTAGTACAATATTTAAGACTGAAGAAGGGAAACTAAGTGTTCTTAAGAAATT
TGGTGGTTTTTTGAGAGGAATTGAGAAGTATAGAATGTTGATCTTTGAGTCAAATCC
TCTGTCTTTTTTGACACCTAGTTACTATGATTCTGATATCATCTTCTTTGTCGGACATG
GAAAAGGAACAATAAGTTTAGTATTTGAAAATAGGAGAGAAAGTTATAACATTGAA
AAGGGACATCTAATGACGATACCAGCAGGAGTAACATTCTACATTATCAACACCGA
CGACAAAAACAAATTGGTGTTGTCCATGTTTCTCATTTCCATTACAAATACCGGTGA
CTTTGTGCCATTTTTCGGGACAGGAGGCAGAAACCCAGAATCATTCCTAAATGCATT
CAGCACTGAAGTTCTTCAAACTTCCTATAATACGTCATATGACAGTCTACAGAAGAT
CTTTGCTCAGAAGAAGGGGGTAATAGTGAAGGCGACTAAAGATCAAATCACAGCTA
TGACAGCTGAAAACGAACAAGAGAAAGAGAAGAACAAATCAACGACGACACAATT
TGGACCGTGTCAAATCTATAGTTATTTGAGGGAGTCTAATGAATATGGGAAACTTTT
TATGGTTGATTATAATGAATATCAGAAACTTCAAGACTTTAACATGTTCATCTCCTTT

AGCAACCTTCAACAGGGTGGCATGAACACTCCGTTTATTAGTTCAAGGGCAACAAA
GATCATAATGGTAGTAGACGGTCGAGGCCGCCTCGAGATGCCGGCCTTAAACGATA
GCATAGATGGCACAACCGAAAACTATTACAAGCAAGTCAGCGCGGAACTAAAGCCG
GGAATGGTGTTTGTCGTTCCTCCCGGCCATCCTATGGTTCTAATGTCTTCTCAGGACG
AAAAACTCGAAACTATTTGCTACGAAGTGAATGCCAAAGGAAATCAAAAGTTTTCC
CTCGCAGGGAAAGTGAACTTGATGAAGAACATACAAAAAGAGGCAAAGGTTTTGTC
ATTTGGAACATTAGCTGAAGAGGTTGACCTTGTGCTCAACAGTCAACAGCAAAATTT
CTTCATAAAAGCTCCTACTTTTAAGTAA (SEQ ID NO: 3)

The encoded amino acid sequence consists of 386 residues, and it is as follows.

MGENGIKNESNTCYVFNDEDFSTIFKTEEGKLSVLKKFGGFLRGIEKYRMLIFESNPLSFL
TPSYYDSDIIFFVGHGKGTISLVFENRRESYNIEKGHLMTIPAGVTFYIINTDDKNKLVLS
MFLISITNTGDFVPFFGTGGRNPESFLNAFSTEVLQTSYNTSYDSLQKIFAQKKGVIVKAT
KDQITAMTAENEQEKEKNKSTTTQFGPCQIYSYLRESNEYGKLFMVDYNEYQKLQDFN
MFISFSNLQQGGMNTPFISSRATKIIMVVDGRGRLEMPALNDSIDGTTENYYKQVSAELK
PGMVFVVPPGHPMVLMSSQDEKLETICYEVNAKGNQKFSLAGKVNLMKNIQKEAKVLS
FGTLAEEVDLVLNSQQQNFFIKAPTFK (SEQ ID NO: 4)

Of the contigs derived from *Silene* mentioned in Example 2, the sequence exhibiting the highest identity with the gene was DN21819, of which the nucleotide sequence of the open reading frame was as follows.

ATGGCTTTGTCAAATGGAAAGGGAGAGAATGGGAAGCCATATGTGTTCAAAGATGA
AGATTTTAGTACATTGTTTGAGACCCAAGAAGGGAGTTTACGAGTTCTTCAGAAATT
TAATCAAAATTCTGATGTTTTAAGAGGAATTCAGAATTATAGGATGTTGATTTTCGA
AGCGAATCCGTTGAGTTTTCTAACTCCTAGTCATTGGGATTCTGATATCATTTTCTTT
GTTGGTCAAGGAAAAGGAACTATCAGTCTGGTTTTTGAAAAGAAGAGAGAAAGCTT
CAACATTGAAAAGGGACATCTCATGACAATACCAGCAGGAGTTACATTCTACATTAT
CAACAGAGATGATACCGATAAATTAGTCCTCTCAATGTTTGTCAGTTCGATTGCTAA
TCCTGGTGATTTTGTGCCATTTTTCACGGCAGGAGGCGGTCAAGACTCTGAATCATT
CCTATCTGCATTCAGCACTGAGGTTCTTGAAGCAGCTTACAATGAATCAAATGAAAC
ACTAAGCAAGATCTTTTCGCAACAGGGGAAGGGGGTGATAGTGAAGGCATCCAAAG
AGCAAATTGAAGCTCTGACAGCTGAAGAAGTGACGACGGAAAGCAATTTCGGGCCT
TGCCAAATCCTTAGTTATCTTCGAGAGTCGAGTGAATGTGGGAGACTTTTCATGGTT
GATTACTGTCAATATAAGAAACTCCAAGACTTCAATATGTTCATCTCCTTTTGCAAC
CTTGAACCGGGATGCATGAATAGTCCATTTATTAGCTCAAGGGCAACAAAGATCAT
AATGGTGGTAAAGGGGCGAGGCCGAGTTGAGATGGCAGCCTCTCGCCCTGGCGAAA

AGGGCTCAATAGAAATTCAGTACATGAAAATCAGCTCGGAATTGAAACCGGGTGTG
GTGTTTGTTATTCCTCCAGGGCACCCTATGGTCCTAATGGCTTCTCAGGATTCAGTTT
TAGAAACTATTTGCTATGAAGTCAATGCTAAAGGAAATCAGAAATTTTCAATTGCAG
GGAAAGTGAACGTGATGAAGAACATTCAGAAAGAAGCAAAAGTACTTACATTTGGA
AAATCAGCTGAAGAAGTTGACAAGGTGCTCGACAGTTCGCAGTCGGATTCTTTCTTG
AAAGCTACTGGTAACAAGTAA (SEQ ID NO: 5)

The corresponding amino acid sequence is as follows. 
MALSNGKGENGKPYVFKDEDFSTLFETQEGSLRVLQKFNQNSDVLRGIQNYRMLIFEAN
PLSFLTPSHWDSDIIFFVGQGKGTISLVFEKKRESFNIEKGHLMTIPAGVTFYIINRDDTDK
LVLSMFVSSIANPGDFVPFFTAGGGQDSESFLSAFSTEVLEAAYNESNETLSKIFSQQGKG
VIVKASKEQIEALTAEEVTTESNFGPCQILSYLRESSECGRLFMVDYCQYKKLQDFNMFI
SFCNLEPGCMNSPFISSRATKIIMVVKGRGRVEMAASRPGEKGSIEIQYMKISSELKPGVV
FVIPPGHPMVLMASQDSVLETICYEVNAKGNQKFSIAGKVNVMKNIQKEAKVLTFGKSA
EEVDKVLDSSQSDSFLKATGNK (SEQ ID NO: 6)
 The identity with SEQ ID NO: 4 is 69%.

The amino acid sequence having the highest identity with Dca32587.1 among those in the protein database deposited with NCBI is KAH9622207.1 of *Heliosperma pusillum* of the family Caryophyllaceae, of which the nucleotide sequence of the open reading frame is as follows.

ATGGCTTTGTTAAATGGAAATGGAGAGTGTGGGAAACCATATGTGTTTAAAGATGA
AGATTTTAGTACATTGTTTAAGACTGAAGAAGGAAGTTTAAGTGTTCTTCAGAAATT
TAATGAAAGTTCTGATGTTTTAAGAGGGATTGAGAATTATAGGATGTTAATCTTTGA
AGCAAATCCATTGAGTTTCTTAACTCCTAGTCATTGGGATTCTGATATCATTTTCTTT
GTTGGTCAAGGAAAAGGAACAATCAGCCTGGTTTTTGAAAAAAAGAGAGAAAGCTT
TAACATTGAAAAGGGACATCTAATGGTTATACCAGCTGGAGTTACATTCTACATTAT
CAACAGAGATGACACTCATAAATTAGTCCTCTCAATGTTTGTTAGTTCTATTGCTAAT
CCTGGTGATTTTGCGCCATTTTTCGGTGCAGGAGGTCAAGACCCTGAATCATTCCTA
TTTGCATTCAGCACTGAGGTTCTTGAAGCAGCTTACAATGCATCAATTAAAACACTA
CAGGGCACCTTTTCGGGGAAGGGAGTGATAGTGAAGGCATCCAAAGAGCAAATTAA
AGCTCTGGCAGCTGAAGAAGTGACGGAAAGCAATTTTGGGCCATGCCAAATCCTTA
GTTATCTTAGAGAGTCGAATGAATGCGGGAGACTCTTCATGGTTGATTACTGTGAAT
ATAAGAAACTCCAAGACTTCAATATGTTCATCTCCTTCTGCAACCTTGAACCGGGAT
GCATGAATAGTCCATTTATTAGTTCAAGGGCAACAAAGATCATAATGGTGGTAAAG
GGGCGAGGTCGAGTGGAGATGGCAGTTTCTCGCAATGGCGAAAAGGGCTCAACAGG
AACTCAATATAAGAAAATCAGCTCTGAATTGAAGCCGGGCGTAGTGTTTGTTATTCC

TCCAGGGCACCCTATGGTCCTTATGGCTTCTCAGGACGAAGTTCTAGAAACTATTTG
CTATGAAGTCAATGCTAAAGGAAATCAGAAGTTTTCACTTGCAGGTAAAGTGAACTT
GATGAAGAACATTCAGAAAGAAGCAAAAGTACTGTCATTTGGAACATCAGCTGAAG
AAGTAGACAGTATGCTTGACAGTCAGCAGTCGGATTTCTTTTTAAAAGCTACTAGTA
ACAAATTACAGGACTAG (SEQ ID NO: 7)

The encoded amino acid sequence is as follows.

MALLNGNGECGKPYVFKDEDFSTLFKTEEGSLSVLQKFNESSDVLRGIENYRMLIFEANP
LSFLTPSHWDSDIIFFVGQGKGTISLVFEKKRESFNIEKGHLMVIPAGVTFYIINRDDTHKL
VLSMFVSSIANPGDFAPFFGAGGQDPESFLFAFSTEVLEAAYNASIKTLQGTFSGKGVIVK
ASKEQIKALAAEEVTESNFGPCQILSYLRESNECGRLFMVDYCEYKKLQDFNMFISFCNL
EPGCMNSPFISSRATKIIMVVKGRGRVEMAVSRNGEKGSTGTQYKKISSELKPGVVFVIP
PGHPMVLMASQDEVLETICYEVNAKGNQKFSLAGKVNLMKNIQKEAKVLSFGTSAEEV
DSMLDSQQSDFFLKATSNKLQD (SEQ ID NO: 8)

The identities for SEQ ID NO: 4 and 6 were 72% and 88%, respectively.

### [Example 10: Measurement of candidate gene activity]

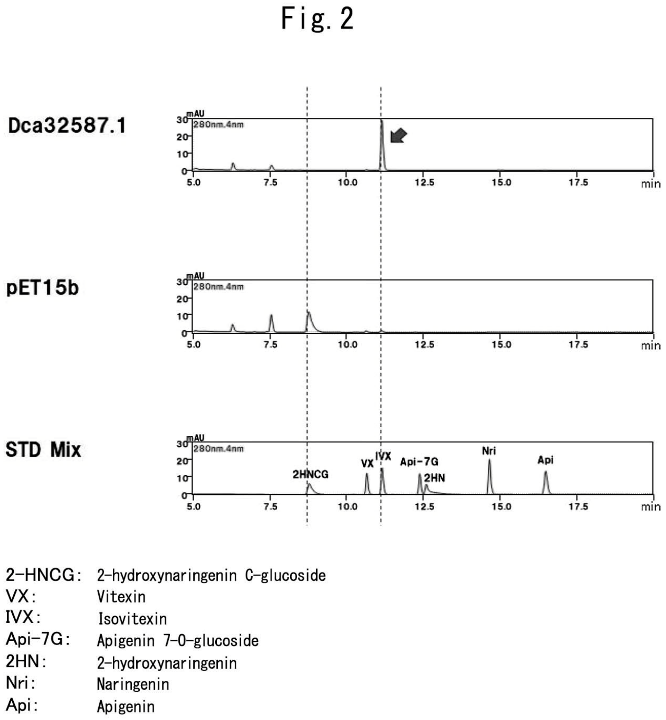

The sequence of Dca32587.1 was expressed by a common method (for example, NPL 11) using *E. coli* expression vector pET15b (Novagen), and the activity was measured. As shown in Fig. 2, *Escherichia coli* expressing Dca32587.1 exhibited activity of specifically converting 2-hydroxynaringenin 6-C-glucoside to isovitexin, and therefore Dca32587.1 was shown to be a gene encoding the desired dehydratase activity.

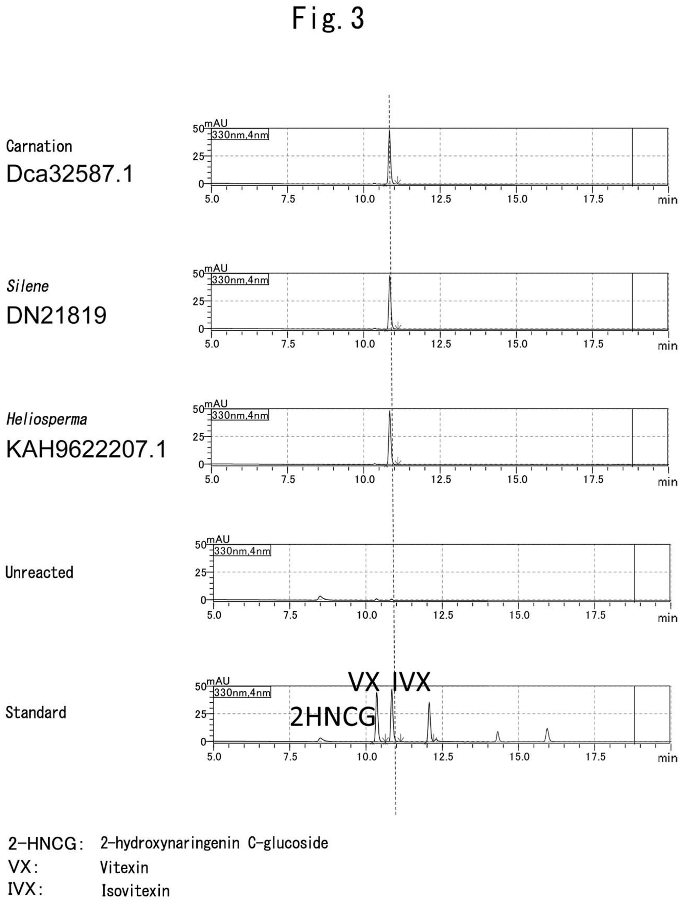

Similarly, when DN21819 of *Silene* and KAH9622207.1 of *Heliosperma pusillum* were expressed using pET15b and the activity was measured, they exhibited activity of specifically converting 2-hydroxynaringenin 6-C-glucoside to isovitexin as shown in Fig. 3, suggesting that these genes encode the target dehydratase activity.

### [Example 11: Acquisition of full-length dehydratase gene of nadeshiko]

As shown in Example 9, the nucleotide sequence of the dehydratase gene of nadeshiko was obtained only as fragments (SEQ ID NO: 1, SEQ ID NO: 2), and therefore the full-length nucleotide sequence was obtained by a common method. Specifically, the database mentioned in Example 6 was searched for the carnation-derived dehydratase gene (SEQ ID NO: 3), and a total of five nadeshiko dehydratase gene fragments were obtained. Since the full length was not obtained even when the fragments were linked, PCR was carried out using cDNA synthesized from nadeshiko mRNA as template, to obtain a nucleotide sequence encoding the entire translation region. The sequence is as follows.

ATGGGTGAAAATGGAGTCAAAATTGTGAGTAACACTTGTTATGTGTTCAATGATGAA
GATTTTAGTACAATGTTTAAGACTGAAGAAGGGAAACTAAGTGTTCTTAAGAAATTT
GGTGGTTTTTTGAGAGGAATTGAGAAGTATAGAATGTTGATCTTTGAATCAAATCCT
CTGTCTTTTTTGACACCTAGTCACTATGATTCTGATATCATCTTCTTTGTTGGACATG
GAAAAGGAACAATAAGTTTAGTATTTGAAAATAGGAGAGAAAGTTACAACATTGAA
AAGGGACATCTAATGACGATACCAGCAGGTGTAACATTTTACATTATCAACACCGA
CGACAAAAACAAATTGATGTTGTCCATGTTTGTCATTTCCATTACAAATACCGGTGA
CTTTGTGCCATTTTTTGGGACAGGAGGTGGAAACCCAGAATCATTCCTAAATGCATT
CAGCACTGAAGTGCTTCAAACTTCCTATAATACGTCATATGACAGTCTACAGAAGAT
CTTTGCTCAGAAGAAGGGGGTGATAGTGAAGGCGACTAAAGATCAAATCACAGCTA
TGACAGCAGAAATCGAACAAGAGAAAGAGAAGAACAAATCAACGACGACACAATT
TGGACCGTGTCAAATCTATAGTTATTTGAGGGAGTCTAATGAATATGGGAAACTTTT
TATGGTTGATTATAATGAATATCAGAAACTTCAAGACTTTAACATGTTCATCTCCTTT
AGCAACCTTCAACAGGGTGGCATGAACACTCCGTTTATTAGTTCAAGGGCAACAAA
GATCATAATGGTAGTAGACGGTCGAGGCCACCTCGAGATGGCGGCCTTAAATGATA
GCATAGATGGCACAACCGAAAACTATTACAAGCAAGTCAGCACGGAACTAAAGCCG
GGTATGGTGTTTGTCGTTCCTCCCGGCCATCCTATGGTTCTAATGTCTTCTCAGGACG
AAAAACTCGAAACTATTTGCTACGAAGTGAATGCCAAAGGAAATCAAAAGTTTTCC
CTCGCAGGGAAAGTGAACTTGATGAAGAACATACAAAAAGAGGCAAAGGTTTTGTC
ATTTGGAACATTAGCTGAAGAGGTTGACCTTGTGCTCAACGGTCAACAGCAAAATTT
CTTCATAAAAGCTCCTACTTTTTAA (SEQ ID NO: 9)

The amino acid sequence encoded by the nucleotide sequence is as follows.

MGENGVKIVSNTCYVFNDEDFSTMFKTEEGKLSVLKKFGGFLRGIEKYRMLIFESNPLSF
LTPSHYDSDIIFFVGHGKGTISLVFENRRESYNIEKGHLMTIPAGVTFYIINTDDKNKLML
SMFVISITNTGDFVPFFGTGGGNPESFLNAFSTEVLQTSYNTSYDSLQKIFAQKKGVIVKA
TKDQITAMTAEIEQEKEKNKSTTTQFGPCQIYSYLRESNEYGKLFMVDYNEYQKLQDFN
MFISFSNLQQGGMNTPFISSRATKIIMVVDGRGHLEMAALNDSIDGTTENYYKQVSTELK
PGMVFVVPPGHPMVLMSSQDEKLETICYEVNAKGNQKFSLAGKVNLMKNIQKEAKVLS
FGTLAEEVDLVLNGQQQNFFIKAPTF*(SEQ ID NO: 10)

The identities for SEQ ID NO: 4, 6 and 8 are 96%, 69% and 71%, respectively.

### [Example 12: Measurement of activity of dehydrogenase gene derived from nadeshiko variety Miite]

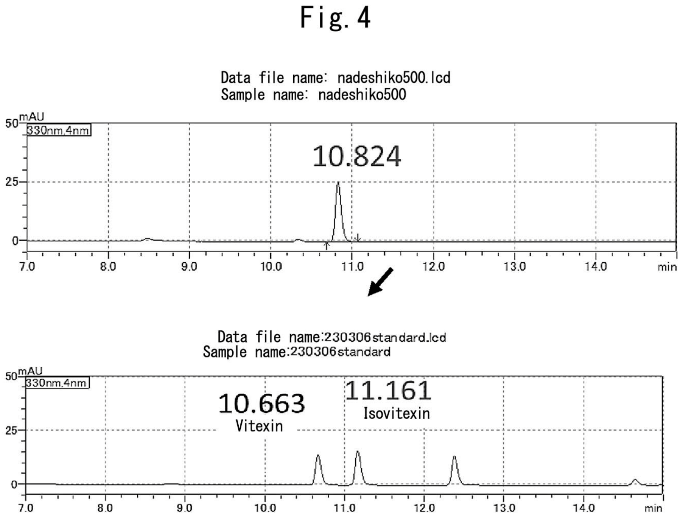

The full-length dehydratase gene of nadeshiko (SEQ ID NO: 9) obtained in Example 11 was expressed as described above using the *E. coli* expression vector pET15b (Novagen), and the activity was measured. Since activity of specifically converting 2-hydroxynaringenin 6-C-glucoside to isovitexin was exhibited, as shown in Fig. 4, the Miite gene was also shown to be a gene coding for the target dehydratase activity (the retention time being shortened during analysis and thus deviating from the standard).

### [Example 13: Acquisition of rose line 7472-2-1]

A binary vector pSPB7472 was constructed containing the bellflower flavonoid 3',5'-hydroxylase, torenia anthocyanin 3',5'-methyl transferase, licorice flavanone 2-hydroxylase, buckwheat flavone C-glucose transferase and *Lotus japonicus* dehydratase genes, and the vector was introduced into Ocean Song (OS) as a rose host, to obtain rose line 7472-2-1 storing 2-hydroxynaringenin 6-C-glucoside in the petals.

### [Example 14: Construction of binary vector]

Based on a common method known to those skilled in the art (see Transgenic Res. 1995, 4, 288-290 and Plant Cell Physiol. 1996, 37, 49-59, for example), binary vectors pSPB8491 and pSPB8493 were fabricated using pBinPLUS as the backbone.
· pSPB8491: El2 35S-carnation 2HDH (SEQ ID NO: 3)-nos terminator (for expression of carnation 2HDH)
· pSPB8493: AtFLS-GUS-nos terminator (GUS gene linked to *Arabidopsis* flavonol synthase gene promoter region, used as a negative control)

### [Example 15: Transient expression of rose petals with carnation 2HDH]

Plasmids pSPB8491 and pSPB8493 were each introduced into *Agrobacterium* Agl0, and colonies were obtained on an L plate containing kanamycin (50 mg/l). After culturing overnight at 28°C with 4 ml of L medium (including kanamycin (50 mg/l)), 2 ml was inoculated into 30 ml of L medium (including kanamycin (50 mg/l)), and culturing was carried out for 5 1/2 hours overnight at 28°C, after which the cells were collected. After washing with 10 ml of MES buffer (10 mM MES-NaOH, pH 5.6, 100 µM acetosyringone, 10 mM MgCl₂), it was suspended in 5 ml of MES buffer.

Six bud petals of OS/7472-2-1 obtained in Example 13 (storing 2-hydroxynaringenin 6-C-glucoside) were prepared by cutting to approximately 5 mm-square on the inner side. These were nicked with a razor and pointed tweezers. They were then placed in the *Agrobacterium* solution and allowed to stand overnight in a dark environment at room temperature. On the following morning, the petals were lightly washed with water, and then one petal each was placed in a 24-well titer plate. After adding 0.5 ml of MES buffering solution to each, the mixture was allowed to stand for 3 nights in an artificial weather chamber (25°, 16 hours, 8 hours of darkness).

After removing the MES buffer, 0.5 ml of acetonitrile was added and the flavonoids of the petals were extracted by shaking (for one night). Vitexin and isovitexin were quantified by HPLC (column: Shim-Pack FC-ODS 4.6 x 150 mm, mobile phase A: 0.1% formic acid aqueous solution, mobile phase B: 0.1% methanol formate solution, flow rate: 1 m1/min, gradient: 0 min → 10% mobile phase B, 12 min → 60% mobile phase B, 20 min → 60% mobile phase B, 21 min → 100% mobile phase B, 22 min, 100% mobile phase B, 23 min → 10% mobile phase B). Although 2-hydroxynaringenin 6-C-glucoside is spontaneously converted to vitexin and isovitexin, since isovitexin is specifically produced when 2HDH acts on 2-hydroxynaringenin 6-C-glucoside, the proportion of isovitexin is expected to increase when 2HDH is functioning.

The numerical values for isovitexin/vitexin in the six petal sections are shown below.

**Table 3]**

| | pSPB8491 (Carnation 2HDH) | pSPB8493 (AtFLS-GUS) |
|---|---|---|
| 1 | 2.42 | 2.22 |
| 2 | 2.29 | 2.23 |
| 3 | 2.38 | 2.11 |
| 4 | 2.28 | 2.15 |
| 5 | 2.29 | 2.19 |
| 6 | 2.33 | 2.19 |

(When a t-test was conducted in both groups, the P value was 0.000492202, which was smaller than 0.01, thus representing a significant difference.)

It was conjectured that in the petals infected with the carnation-derived 2HDH gene, the enzyme from the same gene functioned to produce isovitexin. This demonstrated that the gene functions not only in *E. coli* but also in plants.

[Sequence Listing]

## Claims

1. A polynucleotide encoding an enzyme with dehydrating activity for 2-hydroxyflavanone C-glycoside, wherein the polynucleotide is selected from the group consisting of the following (A) to (E):
(A) polynucleotides consisting of the nucleotide sequences of SEQ ID NO: 3, 5, 7 or 9;
(B) polynucleotides that hybridize with polynucleotides consisting of nucleotide sequences complementary to the nucleotide sequences of SEQ ID NO: 3, 5, 7 or 9 under stringent conditions;
(C) polynucleotides encoding proteins consisting of the amino acid sequences of SEQ ID NO: 4, 6, 8 or 10;
(D) polynucleotides encoding proteins consisting of the amino acid sequences of SEQ ID NO: 4, 6, 8 or 10 having a deletion, substitution, insertion and/or addition of one or more amino acids; and
(E) polynucleotides encoding proteins having amino acid sequences with at least 90% identity to the amino acid sequences of SEQ ID NO: 4, 6, 8 or 10.

2. A polynucleotide according to claim 1, which is a polynucleotide consisting of the nucleotide sequence of SEQ ID NO: 3, 5, 7 or 9.

3. A polynucleotide according to claim 1, which is a polynucleotide encoding a protein consisting of the amino acid sequence of SEQ ID NO: 4, 6, 8 or 10.

4. A protein encoded by a polynucleotide according to any one of claims 1 to 3.

5. A vector that comprises a polynucleotide according to any one of claims 1 to 3.

6. A transgenic plant that comprises a polynucleotide according to any one of claims 1 to 3, or an inbred or outbred progeny of the same.

7. Propagules, partial plant bodies, tissue or cells of a transgenic plant according to claim 6, or its inbred or outbred progeny.

8. Cut flowers of a transgenic plant according to claim 6, or its inbred or outbred progeny, or a processed form created from the cut flowers.

9. A method for creating a transgenic plant,
the method comprising a step of introducing into plant cells a polynucleotide according to any one of claims 1 to 3.

10. A non-human host that comprises a polynucleotide according to any one of claims 1 to 3.

11. A method for producing an enzyme with dehydrating activity for 2-hydroxyflavanone C-glycoside, the method comprising:
a step of culturing a non-human host according to claim 10, and
a step of harvesting from the non-human host an enzyme with dehydrating activity for 2-hydroxyflavanone C-glycoside.

12. A method for producing flavone C-glycoside,
wherein the method includes contacting an enzyme produced by the method according to claim 11 with 2-hydroxyflavanone C-glycoside.
